# FASCICULE DE BREVET EUROPEEN

(11) **EP 0 472 482 B1**
(45) Date de publication et mention de la délivrance du brevet: **06.11.1996**
(21) Numéro de dépôt: 91450009.5
(22) Date de dépôt: 07.08.1991
(51) Int. Cl.: C12Q 1/68

(54) **Nouveau procédé de dosage d'acide désoxyribonucleique présent en position extracellulaire dans un milieu**
Neues Verfahren zur quantitativen Bestimmung von extrazellularer DNS in einer biologischen Flüssigkeit
New procedure for the quantitative determination of extracellular DNA in a biological fluid

(30) Priorité: 21.08.1990 FR 9010700
(43) Date de publication de la demande: 26.02.1992
(73) Titulaire: SOCIETE FRANCAISE DE RECHERCHES ET D'INVESTISSEMENT (SFRI),Societé Anonyme, 33127 Saint Jean D'Illac (FR)
(72) Inventeur: Fournie, Gilbert, F-31500 Toulouse (FR)
(74) Mandataire: Thébault, Jean-Louis

(56) Documents cités:
- WO-A-87/02064
- WO-A-88/06633
- JOURNAL OF IMMUNOLOGICAL METHODS, vol. 63, no. 3, 28 octobre 1983, Elsevier Science Publishers B.V.; R.L. RUBIN et al., pp. 359-366/
- VIROLOGY, vol. 126, 1982, Academic Press Inc.; D.J. BRIGATI et al., pp. 32-50/
- ONCOLOGY, vol. 47, mai-juin 1990, S. Karger AG, Basel (CH); Y. MAEHARA et al., pp. 282-286/

## Description

La présente invention concerne un nouveau procédé de dosage de microquantités d'acide désoxyribonucléique (ADN) présent en position extracellulaire dans un milieu liquide.

Un tel dosage présente un grand intérêt dans de nombreuses circonstances :
1. D'un point de vue physiologique, la présence d'ADN en position extracellulaire dans un organisme vivant peut dépendre de deux types de phénomènes. D'une part, la mort cellulaire est un phénomène normal et permanent qui entraine la libération dans le milieu extracellulaire de produits de dégradation de la chromatine (et donc d'ADN) et dont le rôle dans les phénomènes de développement de l'organisme et de maturation de divers systèmes fonctionnels (en particulier, système immunitaire) est maintenant bien établi. D'autre part, certains auteurs ont émis l'hypothèse que la libération d'ADN dans le secteur extracellulaire dépendrait d'un processus cellulaire actif d'excrétion et représenterait un mécanisme de communication intercellulaire.
2. D'un point de vue pathologique, tout processus s'accompagnant de phénomènes de destruction d'une structure vivante contenant de l'ADN, qu'il s'agisse de cellules d'un organisme pluricellulaire ou de particules infectieuses (bactériennes, virales ou parasitaires) se traduit par la libération dans le milieu extracellulaire avoisinant d'ADN (et/ou d'ARN). La signification de ce phénomène, l'intérêt de son étude et les méthodologies mises en oeuvre pour l'explorer ne sont pas univoques (Fournié, Eurorhumatology, Tagas and son press, Athens, 1987, pp 9-13) :
   A/ Au cours de certaines affections, en particulier infectieuses ou héréditaires, de l'ADN spécifique d'un agent pathogène on d'un processus pathogène peut être présent dans un liquide extracellulaire. La détection et l'identification de cet ADN peut alors revêtir un intérêt étiologique. Dans ces situations, il est fait le plus souvent appel à la technique d'hybridation. Le principe de cette technique est de mettre en présence l'ADN éventuellement présent dans l'échantillon à tester (préalablement purifié et dénaturé par la chaleur et fixé sur un support solide) et une sonde d'ADN complémentaire préalablement marquée soit par une substance radioactive soit par un marqueur froid, comme par exemple la biotine. L'immobilisation de l'ADN sur un support solide se fait le plus souvent sur membrane (nylon ou nitrocellulose par exemple). Cette méthode est relativement longue et n'est que semi-quantitative, mais permet de détecter de l'ADN spécifique au niveau du picogramme, même en utilisant un marquage "froid" de l'ADN (Barraud-Hadidane et al, Arch Toxicol, 1987, Suppl 11, 200-205). Une technique quantitative utilisant comme support solide une plaque de microtitration et une sonde d'ADN marquée à la biotine a également été décrite par Nagata et al (FEBS, 1985, 183, 379-382).
   B/ A l'opposé, dans de nombreuses circonstances, il peut être important de détecter et de doser de l'ADN indépendemment de sa spécificité, i.e. des caractéristiques moléculaires de sa structure primaire :
      1/ En pathologie humaine, le dosage de l'ADN peut revêtir suivant les circonstances, soit un intérêt diagnostique et/ou pronostique, soit un intérêt physiopathologique :
         a/ La libération d'ADN dans le secteur extracellulaire est le reflet et le témoin de processus de mort cellulaire et peut permettre de déceler et de quantifier cette mort cellulaire in vivo dans l'organisme entier. La détection et le dosage de l'ADN peut donc, dans diverses situations de pathologie expérimentale ou humaine, revêtir un intérêt diagnostique ou pronostique. C'est le cas par exemple de la maladie thromboembolique (Vargo et al, Chest, 1990, 97, 63-68) ou des vascularites (Steinman, Arthritis Rheum, 1982, 25, 1425-1430).
         b/ Il est possible que l'ADN circulant puisse jouer un rôle pathogène. Différents mécanismes peuvent être mis en jeu : (i) du fait de ses propriétés physicochimiques, en particulier de sa viscosité et de sa charge électrique, l'ADN circulant peut perturber le fonctionnement normal de la circulation et/ou se déposer de manière préférentielle dans certains tissus ou organes ; (ii) l'ADN extracellulaire peut activer divers systèmes biologiques, tels les systèmes du complément et de la coagulation ; (iii) il peut également jouer un rôle pathogène du fait de sa nature et de ses propriétés "géniques" en s'intégrant au génome de l'hôte ; (iv) enfin il peut être à l'origine de la formation de complexes macromoléculaires en présence d'autoanticorps, mécanisme impliqué dans le développement des lésions glomérulaires chez les patients atteints de lupus érythémateux disséminé (Fournié, Kidney Int, 1988, 33, 487-497).
      2/ En pharmaco-toxicologie, le dosage de l'ADN circulant peut permettre de déceler in vivo la cytotoxicité d'une substance médicamenteuse ou toxique (Bret et al, Toxicology, 1990, 61, 283-292). Il pourrait donc être utile dans la phase de développement d'un médicament, dans la sélection, au sein d'une même famille pharmacologique, de la substance douée du meilleur rapport efficacité/toxicité. Par ailleurs, il pourrait être utilisé au cours des essais cliniques de phase I chez l'homme pour déceler plus précocement une toxicité médicamenteuse non prévisible à partir des données pharmacotoxicologiques expérimentales. Enfin le dosage de l'ADN présent en position extracellulaire dans divers liquides biologiques dont en particulier les urines, pourrait être utile en pratique courante pour le dépistage des effets toxiques de différents xénobiotiques et médicaments.
      3/ Dans le domaine de l'étude de la biocompatibilité de divers systèmes de circulation extracorporelle utilisés en pratique médicale, en particulier des systèmes d'hémodialyse, le dosage de l'ADN permet d'analyser in vivo l'intensité des phénomènes de mort cellulaire liés à l'éventuelle bio-incompatibilité du système (Fournié et al, Amer J Nephrol, 1989, 9, 384-391).
      4/ Dans le domaine des biotechnologies, le dosage d'ADN résiduel, contaminant les milieux contenant des substances biologiques actives obtenus par exemple par génie génétique est important pour en contrôler l'inocuité (WHO expert commitee on biological standardisation, 1982).

Compte tenu des intérêts que présente le dosage de l'ADN circulant et de l'ADN présent en position extracellulaire dans divers liquides biologiques, différentes techniques colorimétriques, fluorimétriques, immunologiques et biologiques ont été développées. Ces techniques présentent des limites en particulier lorsque l'ADN doit être dosé dans des liquides biologiques complexes tels le plasma. Ces limites concernent la spécificité, la sensibilité et le caractère quantitatif des dosages d'une part, la nécessité d'un traitement préalable de l'échantillon dans lequel le dosage doit être effectué d'autre part :
1/ La spécificité de certaines techniques n'est pas assurée du fait des interactions entre diverses substances autres que l'ADN présentes dans l'échantillon à doser et les réactifs utilisés dans la méthode de dosage. C'est le cas des techniques fluorimétriques et colorimétriques, mais également des techniques immunologiques et des méthodes utilisant des substances fixant l'ADN. Le contrôle de la spécificité du dosage impose le recours systématique à un dosage complémentaire après digestion de l'ADN dosé à l'aide d'un enzyme spécifique tel la désoxyribonucléase I (Steinman, J Clin Invest, 1975, 56, 512-515).
2/ Les limitations de sensibilité et l'absence de caractère quantitatif des techniques sont liées à divers facteurs : (i) le principe même de certaines techniques, (e.g. la contreimmunoélectrophorèse) ne permet pas dans certains cas un dosage quantitatif ; (ii) la taille variable des molécules d'ADN à doser : le nombre de molécules d'ADN de tailles différentes est variable pour une même quantité, alors que le signal décelé est fonction du nombre de molécules d'ADN réactives (et non pas de la quantité absolu d'ADN telle que l'on peut l'exprimer en nombre de nucléotides ou de paires de bases) ; (iii) les pertes d'ADN liées aux faibles rendements lorsque les quantités d'ADN à purifier sont faibles.
3/ Le traitement de l'échantillon avant dosage, pour purifier et éventuellement concentrer l'ADN nécessaire dans certaines techniques, est souvent laborieux et parfois aléatoire, entraînant comme précédemment mentionné des limitations dans la récupération quantitative de l'ADN présent dans l'échantillon de départ, à moins de recourir à des méthodes difficiles à mettre en oeuvre en pratique, telle l'ultracentrifugation (Shapiro, Anal Biochem, 1881, 110, 229-231).

Pour surmonter ces limites et difficultés, il a été proposé de nouvelles méthodes de récupération et de purification de l'ADN et l'utilisation de méthodes et de matériels issus des techniques de biologie moléculaire pour réaliser un dosage quantitatif de l'ADN.
1/ Raptis et Ménard (J Clin Invest 1980, 66, 1391-1399) ont été les premiers à réaliser une détection de l'ADN circulant chez des patients atteints de lupus érythémateux disséminé après l'avoir purifié et marqué in vitro par un isotope radioactif en utilisant la technique de déplacement de coupure haplotomique ("Nick translation") elle même décrite précédemmnt par Rigby et collaborateurs (J Mol Biol 1977, 113, 237-251). La lourdeur des techniques de préparation du matériel à doser (dilution de l'échantillon, digestion enzymatique, extraction organique, dialyse, purification sur colonne, précipitation à l'éthanol) ne permettait pas d'appliquer cette méthode à la détection et au dosage de l'ADN en pratique courante.
2/ Fournié et al (Anal Biochem 1986, 158, 250-256) ont décrit un nouveau procédé de récupération et de dosage de microquantités d'ADN dans un liquide biologique acellulaire. Cette méthode basée sur l'extraction d'ADN par un traitement au phénol, sa récupération par précipitation à l'éthanol en utilisant la gélatine comme agent coprécipitant et son dosage quantitatif à l'aide de la technique de déplacement de coupure haplotomique a permis de résoudre le problème d'un traitement simultané de plusieurs échantillons compatible avec l'utilisation de cette méthode dans certaines applications en pratique. Les limitations de cette méthode sont : (i) la nécessité d'extraction de l'échantillon avant son dosage qui impose plusieurs étapes de centrifugation à des températures différentes, une étape de lyophilisation ou d'évaporation sous vide et une étape de redissolution de l'échantillon ; (ii) le recours à un marquage radioactif de l'ADN ; (iii) les étapes de lavages des filtres sur lesquels ont été déposés les échantillons pour séparer l'ADN radiomarqué du nucléotide libre marqué au tritium (étapes qui peuvent difficilement être utilisées en cas de marquage d'ADN par la biotine ou tout autre marqueur "froid" du fait de la difficulté à éluer l'ADN adsorbé sur le filtre).
3/ Briggs et al (IBL Juin 1989 pp 18-25) ont décrit un "total DNA assay system" qui autorise un dosage enzymatique de l'ADN au niveau du picogramme (2-200 pg). Selon cette méthode l'ADN purifié et dénaturé par la chaleur, est fixé sur une membrane par l'intermédiaire d'une protéine fixant l'ADN monocaténaire, et est quantifié à l'aide d'un anticorps monoclonal anti-ADN monocaténaire couplé à un enzyme (uréase) à l'aide d'un biocapteur potentiométrique développé par Molecular Devices Corp (Menlo Park, Californie, USA). Les limitations de cette méthode sont multiples, en particulier : (i) l'utilisation d'un appareillage particulier et couteux (>200 000 FF) pour réaliser le dosage ; (ii) le caractère non quantitatif, le "rendement" de dosage étant fonction de la taille des molécules d'ADN ; (iii) la nécessité de réaliser un prétraitement "adapté" à chaque type d'échantillon (dénaturation de l'ADN à la chaleur, préfiltration sur nitrocellulose et digestion à la protéinase K suivi éventuellement d'une séparation en fonction de la taille de l'ADN).

Ces deux dernières techniques peuvent être mises en oeuvre sur de faibles volumes et permettent de doser l'ADN extracellulaire indépendemment de sa nature au niveau du picogramme, mais elles sont inutilisables pour le but que s'est fixé la présente invention, à savoir le dosage de quantités d'ADN de l'ordre de quelques picogrammes dans des liquides biologiques complexes (i) ne nécessitant pas d'extraction préalable de l'ADN avant dosage, (ii) ne nécessitant pas le recours à des isotopes radiactifs, (iii) réalisable sur support solide, en particulier sur plaque de microtitration, (iv) permettant d'obtenir le résultat en moins de deux heures.

La présente invention a pour but de pourvoir à un procédé de dosage de l'ADN qui répond mieux aux nécessités de la pratique que les procédés proposés dans l'Art antérieur. En effet, le nouveau procédé conforme à la présente invention :
- est réalisable sur un support solide conventionnel type "plaque de microtitration", sur lequel sont effectuées les différentes réactions nécessaires à la réalisation du dosage,
- ne nécessite pas d'extraction de l'ADN avant les étapes de dosage proprement dit, en particulier lorsque les échantillons sont des prélèvements de plasma sanguin,
- ne nécessite pas le recours à des substances radioactives,
- ne nécessite pas d'appareillage spécifique, la lecture du signal pouvant se faire à l'aide d'un spectrophotomètre, d'un spectrofluorimètre ou d'un biocapteur conventionnel,
- ne requiert que quelques microlitres d'échantillon,
- est spécifique de l'ADN,
- est d'une grande sensibilité,
- peut être mis en oeuvre simultanément sur plus de cent échantillons
- et permet d'obtenir le résultat du dosage en moins de deux heures

La présente invention a pour objet un procédé de dosage de microquantités d'ADN présentes en position extracelluaire dans un liquide biologique, en particulier le plasma sanguin, qui est caractérisé en ce qu'il comporte les étapes suivantes:
- une étape d'adsorption de l'ADN à doser sur un support solide,
- une étape de marquage de cet ADN par incorporation de un ou plusieurs désoxyribonucléotides triphosphates marqués mettant en jeu un ou des enzymes spécifiques de l'ADN,
- une étape de dosage de l'ADN par toute méthode appropriée révélant le marqueur incorporé à l'ADN, ces étapes pouvant être séparées les unes des autres par des étapes de lavages.

Selon un mode de mise en oeuvre préféré du procédé conforme à la présente invention, l'adsorption de l'ADN sur le support solide se fait par l'intermédiaire de substances constitués par la gélatine ou de complexes macromoléculaires cationiques présentant une forte affinité pour l'ADN.

Selon une modalité avantageuse de ce mode de mise en oeuvre, les substances utilisées pour adsorber l'ADN sont représentées par une ou des protéines histoniques.

Selon une disposition avantageuse de ce mode de mise en oeuvre, le support solide est sensibilisé par incubation d'un mélange de protéines histoniques en tampon carbonate 0,05-0,1M pH environ 9,6, plusieurs heures, quelle que soit la température d'incubation.

Selon un mode de mise en oeuvre préféré du procédé conforme à la présente invention, l'adsorption de l'ADN présent dans l'échantillon est réalisée au cours d'une incubation de quelques minutes à plusieurs heures quelle que soit la température d'incubation.

Selon un mode de mise en oeuvre préféré du procédé conforme à la présente invention, le support solide subit un ou plusieurs lavages après adsorption de l'ADN.

Selon une modalité avantageuse de ce mode de mise en oeuvre, le ou les lavages du support solide sont effectués en tampon trishydroxyaminométhane 2,5 mM pH8, EDTA 1mM.

Selon une autre modalité avantageuse de ce mode de mise en oeuvre, le ou les lavages du support solide sont effectués en tampon Borate (force ionique environ 0,1), 0,1 % Tween® 20, pH 8-8,5 (Tween étant une marque déposée)

Selon un mode de mise en oeuvre préféré du procédé conforme à la présente invention, le marquage de l'ADN adsorbé est réalisé à l'aide d'une réaction de biologie moléculaire mettant en jeu un ou plusieurs enzymes spécifiques de l'ADN permettant l'incorporation d'au moins un désoxyribonucléotide triphosphate marqué chimiquement par une substance pouvant être ultérieurement révélée, par exemple à l'aide d'une réaction enzymatique.

Selon une disposition avantageuse de ce mode de mise en oeuvre, le marquage de l'ADN est réalisé à l'aide d'une réaction de déplacement de coupure haplotomique ("Nick translation") par incorporation d'au moins un désoxyribonucléotide triphosphate marqué chimiquement par une substance pouvant être ultérieurement révélée, par exemple à l'aide d'une réaction enzymatique.

Selon une modalité avantageuse de ce mode de mise en oeuvre, le marquage au cours de la deuxième étape peut être réalisé au cours d'une incubation de quelques minutes à plusieurs heures, en particulier de 15 à 240 minutes (par exemple 60 minutes) à température de 15 à 40°Celsius environ (par exemple 30°Celsius).

Selon une disposition avantageuse de ce mode de mise en oeuvre, le ou les nucléotides incorporés dans l'ADN sont marqués par la biotine et l'enzyme utilisé pour sa ou leur détection est lié à l'avidine ou à la streptavidine.

Selon une disposition avantageuse de ce mode de mise en oeuvre du procédé conforme à la présente invention, la marquage par déplacement de coupure haplotomique est effectué par incubation de l'ADN adsorbé sur le support solide avec 10 à 100 picomoles d'un ou plusieurs désoxyribonucléotides triphosphates marqués, éventuellement environ 10 à 100 picomoles de désoxyribonucléotides triphosphates non marqués, environ 0,04 unités d'ADN polymérase et environ 4 picogrammes de désoxyribonucléase I, en présence d'un tampon tel que le trishydroxyaminométhane (0,5-5mM), de chlorure de magnésium (2-5mM), et éventuellement de bêta mercaptoéthanol (7-15mM), et d'albumine bovine sérique (20-60 microgrammes/ml).

Selon un mode de mise en oeuvre préféré du procédé conforme à la présente invention, le support solide subit un ou plusieurs lavages avant la révélation du marqueur incorporé quantitativement dans l'ADN.

Selon une modalité avantageuse de ce mode de mise en oeuvre, le ou les lavages éventuels sont effectués en tampon Phosphate 0,2M Citrate 0,1M pH 5,4 Tween 20 0,1%.

Selon une autre modalité avantageuse de ce mode de mise en oeuvre, le ou les lavages du support solide sont effectués en tampon Borate (force ionique environ 0,1), 0,1 % Tween 20, pH 8-8,5.

Selon un mode de mise en oeuvre préféré du procédé conforme à la présente invention, la révélation du marqueur incorporé dans l'ADN se fait à l'aide d'une réaction enzymatique en utilisant un substrat dont la réaction en présence de l'enzyme correspondant est responsable de l'émission d'un signal quantifiable à l'aide de l'appareillage convenable.

Selon une modalité avantageuse de ce mode de mise en oeuvre, le marqueur incorporé à l'ADN étant la biotine, la révélation de ce marqueur incorporé dans l'ADN se fait par addition au cours d'une troisième étape d'un composé avidine/enzyme ou streptavidine/enzyme.

Selon une modalité avantageuse de ce mode de mise en oeuvre, l'enzyme couplé à l'avidine ou à la streptavidine est la peroxydase.

Selon une modalité avantageuse de ce mode de mise en oeuvre, le complexe streptavine/peroxydase préalablement dilué à la concentration adéquate en tampon Phosphate 0,2M Citrate 0,1M pH 5,4 4% polyéthylène glycol (6000) est incubé 5 à 30 minutes à température d'environ 4 à 37°Celsius.

Selon un mode de mise en oeuvre préféré du procédé conforme à la présente invention, l'activité enzymatique de la peroxydase est déterminée quantitativement après lavages du support solide par addition d'un substrat dont la modification sous l'effet de l'enzyme donnera un signal mesurable.

Selon une modalité avantageuse de ce mode de mise en oeuvre, le ou les lavages éventuels sont effectués en tampon Phosphate 0,2M Citrate 0,1M pH 5,4 Tween 20 0,1%.

Selon une autre modalité avantageuse de ce mode de mise en oeuvre, le ou les lavages du support solide sont effectués en tampon Borate (force ionique environ 0,1), 0,1 % Tween 20, pH 8-8,5.

Selon une modalité avantageuse de ce mode de mise en oeuvre, l'activité enzymatique de la peroxydase est déterminée quantitativement après des lavages du support solide en tampon Phosphate 0,2M Citrate 0,1M pH 5,4 Tween 20 0,1% par addition de 3 3′ 5 5′ tétraméthylbenzidine, et la lecture de la réaction colorée est effectuée quelques minutes plus tard après arrêt de la réaction par addition d'acide sulfurique 2-4N à l'aide d'un spectrophotomètre à 450 nm.

Outre les dispositions qui précèdent, l'invention comprend encore d'autres dispositions qui ressortiront de la description qui va suivre.

La présente invention vise plus particulièrement (i) le nouveau procédé de dosage de l'ADN présent dans un liquide biologique en position extracellulaire, conforme aux dispositions qui précèdent, (ii) les analyses biologiques utilisant ce procédé, ainsi que (iii) les situations physiologiques et pathologiques élucidées et (iv) les diagnostics établis grâce au procédé conforme à la présente invention.

L'invention sera mieux comprise à l'aide du complément de description qui va suivre, qui se réfère à des exemples de mise en oeuvre et d'application du procédé objet de la présente invention. Il doit être bien entendu, toutefois, que ces exemples sont donnés uniquement à titre d'illustration de l'objet d'invention, dont ils ne constituent en aucune manière une limitation.

### Exemple 1 : détection d'ADN circulant dans le plasma

- sensibilisation de la plaque de microtitration : 50 µl de protéines histoniques à 10 µg/ml en tampon carbonate 0,1M pH 9,6. Incubation 24 heures à 4°C suivie de trois lavages en tampon Borate (0,1 de force ionique) 0,1% Tween 20.
- adsorption de l'ADN sur le support solide : incubation de 50 µl de plasma 15 minutes à 30°C.
- marquage de l'ADN : après 3 lavages en tampon trishydroxyaminométhane 2,5mM pH8 1mM EDTA (acide éthylènediamine tétraacétique), addition de 50 µl de milieu réactionel 0,5 mM Trishydroxyaminométhane 0,1M pH8, 3mM MgCl₂, 50 µg/ml BSA, 9 mM bêta 2 mercaptoéthanol et contenant 40 picoMoles de biotine 14-dATP, 20 picoMoles de dGTP, dTTP et dCTP, 0,04 unités de DNA polymérase et 4 picogrammes de désoxyribonucléase pancréatique I, et incubation 1 heure à 30°C.
- révélation du marquage : après trois lavages en tampon Phosphate 0,2M/Citrate 0,1M pH 5,4 Tween 20 0,1%, addition de 40 µl de conjugué streptavidine-peroxydase (Amersham) dilué au 1/1000 en tampon Phosphate/Citrate 4% polyéthylèneglycol. Après 15 minutes d'incubation à 30°C et quatre lavages en tampon Phosphate 0,2M/Citrate 0,1M pH5,4 Tween 20 0,1%, addition de 50 µl de substrat (3 3' 5 5' tétraméthylbenzidine). Après quelques minutes, la réaction colorée est arrêtée par addition de 50 µl de H₂SO₄ 4N et la lecture est effectuée à 450 nm dans un spectrophotomètre "Multiscan® Flow".

Exemple 2 : détection d'ADN circulant dans le plasma d'une souris injecté par endotoxine bactérienne (ou lipopolysaccharide : LPS) et dans le plasma d'une souris normale, en utilisant comme support solide une plaque de microtitration sensibilisée par des protéines histoniques. (Tableau I).
- sensibilisation de la plaque de microtitration : 50 µl de protéines histoniques à 10 µg/ml en tampon carbonate 0,1M pH 9,6. Incubation 24 heures à 4°C suivie de trois lavages en tampon Borate (0,1 de force ionique) 0,1% Tween 20, pH 8,3.
- adsorption de l'ADN sur le support solide : incubation de 35 µl de plasma de souris à différentes dilutions, 120 minutes à 30°C.
- marquage de l'ADN : après 3 lavages en tampon trishydroxyaminomérhane 2,5mM pH8 1mM EDTA (acide éthylènediamine tétraacétique), addition de 35 µl de milieu réactionel 0,5 mM Trishydroxyaminométhane 0,1M pH8, 3mM MgCl₂, 50 µg/ml BSA, 9mM bêta 2 mercaptoéthanol et contenant 40 picoMoles de biotine 14-dATP, 20 picoMoles de dGTP, dTTP et dCTP, 0,04 unités de DNA polymérase et 4 picogrammes de désoxyribonucléase pancréatique I, et incubation 2 heures à 30°C.
- révélation du marquage : après trois lavages en tampon Phosphate 0,2M/Citrate 0,1M pH5,4 Tween 20 0,1%, addition de 40 µl de conjugué streptavidine-peroxydase (BRL) dilué au 1/1000 en tampon Phosphate/citrate 4% polyéthylèneglycol. Après 30 minutes d'incubation à 30°C et quatre lavages en tampon Phosphate 0,2M/Citrate 0,1M pH5,4 Tween 20 0,1%, addition de 50 µl de substrat (3 3' 5 5' tétraméthylbenzidine). Après quelques minutes, la réction colorée est arrêtée par addition de 50 µl de H₂SO₄ 4N et la lecture est effectuée à 450 nm dans un spectrophotomètre "Multiscan Flow".

**Tableau I**

| Détection d'ADN circulant dans le plasma d'une souris injectée par LPS et dans le plasma d'une souris normale | | | |
|---|---|---|---|
| | | Unités DO¹ (DOx1000) | |
| | dilution | puits sensibilisés par histones | puits non sensibilisés |
| plasma "LPS"² | 1/3 | 1462 | 128 |
| | 1/15 | 972 | 90 |
| | | | |
| plasma normal³ | 1/3 | 116 | 122 |
| 1/15 | 101 | 103 | |

| | | | |
|---|---|---|---|
| 1 : DO = densité optique | | | |
| 2 : plasma de souris femelle OF1 agée de huit semaines injectée huit heures avant la saignée par voie intrapéritonéale par 100 microgrammes de LPS de Salmonella minnesota Re 595 dilué dans 0,15M NaCl (0,2 ml) | | | |
| 3 : souris femelle OF1 agée de huit semaines injectée huit heures avant la saignée par 0,2 ml de 15M NaCl. | | | |

Exemple 3 : détection d'ADN circulant dans le plasma d'une souris injectée par endotoxine bactérienne dosé à différentes dilution dans du plasma de souris normale, en utilisant comme support solide une plaque de microtitration sensibilisée par des protéines histoniques. (Tableau II).

La méthodologie est identique à celle décrite dans l'exemple 2. Les résultats obtenus ont été corrigés pour la valeur obtenue en DO dans le plasma de la souris normale.

**Tableau II**

| Effet des dilutions | |
|---|---|
| dilution¹ | DO² |
| 1/5,6 | 1412 |
| 1/22 | 1063 |
| 1/90 | 523 |
| 1/360 | 192 |
| 1/1400 | 76 |
| 1/5700 | 26 |

| | |
|---|---|
| 1 : dilution du plasma de souris injectée par endotoxine bactérienne (LPS) dans du plasma de souris normale (NMP). | |
| 2 : DO : densité optique. Les résultats sont exprimés par la différence en unités DO (DOx1000) entre la valeur donnée par le plasma de souris injectée par LPS dilué en NMP et celle donnée par le NMP. | |

### Exemple 4 : dosage d'ADN d'origines différentes. (Tableau III).

La méthodologie utilisée pour doser des quantités identiques d'ADN d'origines différentes est identique à celle utilisée dans les exemples 2 et 3.

Les dosages ont été effectués sur 35 µl d'ADN à 731 ng/ml de 4 origines différentes :

**Tableau III**

| Dosage d'ADN de différentes origines | |
|---|---|
| ADN¹ | unités DO |
| A | 1127 |
| B | 1007 |
| C | 991 |
| D | 1195 |
| /² | 182 |

| | |
|---|---|
| 1 : Origine des ADN dosés : A : bactériophage (MP2, Boerhinger Mannheim) B : plasmide (PBR 322, Boerhinger Mannheim) C : thymus de veau (type V Sigma) D : mononucléosome extraits de cellules de hamster CHO | |
| 2 : / = contrôle négatif (NaCl 0,15M) | |

Exemple 5 : dosage de l'ADN dans du plasma de souris injectée par LPS dilué au 1/4 dans du plasma normal de souris(NMP). Effet des temps des étapes d'adsorption et de marquage. Contrôle de la spécificité du dosage. ( Tableau IV).

La méthodologie utilisée est décrite dans l'exemple 1. Deux temps d'adsorption (15 et 60 minutes) et quatre temps de marquage (15, 30, 60, 120 minutes) ont été testés. La spécificité du dosage a été contrôlée en omettant les enzymes spécifiques de l'ADN dans le milieu réactionnel de marquage de l'ADN.

**Tableau IV**

| Effet des temps des étapes d'adsorption et de marquage et contrôle de la spécificité du dosage. | | | | | |
|---|---|---|---|---|---|
| | | Unités DO (DOx1000) | | | |
| durée de l'étape (minutes) | | plasma de souris injectée par LPS | | NMP | |
| adsorption | marquage | +¹ | -² | + | - |
| 15 | 15 | 938 | 188 | 232 | 199 |
| 15 | 30 | 1141 | 212 | 242 | 214 |
| 15 | 60 | 1453 | 219 | 246 | 203 |
| 15 | 120 | 1478 | 224 | 262 | 212 |
| | | | | | |
| 60 | 15 | 961 | 114 | 160 | 122 |
| 60 | 30 | 1064 | 116 | 149 | 111 |
| 60 | 60 | 1387 | 107 | 162 | 99 |
| 60 | 120 | 1444 | 190 | 276 | 180 |

| | | | | | |
|---|---|---|---|---|---|
| 1 : + : enzymes spécifiques de l'ADN présents. | | | | | |
| 2 : (-) : enzymes spécifiques de l'ADN absents. | | | | | |

Il ressort des exemples d'application qui précèdent que le procédé qui fait l'objet de la présente invention permet de doser quantitativement l'ADN dans le plasma et d'une manière générale dans un milieu biologique contenant de l'ordre de 1000 fois plus de protéines que d'ADN et constitue un procédé facile à mettre en oeuvre ne requérant qu'un appareillage pour la lecture en densité optique dans le spectre visible. Le procédé conforme à la présente invention est reproductible et suffisamment sensible pour détecter de l'ADN dans des échantillons de quelques microlitres à des concentrations de l'ordre du nanogramme par millilitre.

En outre, le procédé conforme à la présente invention est plus rapide et permet de doser simultanément au moins 10 fois plus d'échantillons que les procédés proposés dans l'art antérieur.

Le procédé conforme à la présente invention devrait en raison des avantages qu'il présente, dont certains ont été précisés dans ce qui précède, permettre une meilleure comphéhension de la signification physiologique et physiopathologique de l'augmentation des taux d'ADN plasmatique chez l'homme aussi bien que dans des conditions expérimentales et devrait être un outil de grande valeur pour l'étude de toute situation où il est en pratique important de quantifier les phénomènes de mort cellulaire ou de détecter la contamination d'un échantillon biologique par de l'ADN.

## Revendications

1. Procédé de dosage de d'ADN présent en position extracellulaire dans un milieu, caractérisé en ce qu'il comprend les étapes suivantes :
- fixation de l'ADN présent dans le milieu par adsorption sur un support solide,
- incorporation dans l'ADN fixé d'au moins un désoxyribonucléotide marqué pour marquer ledit ADN fixé, et
- révélation directe ou indirecte du marqueur incorporé audit ADN fixé.

2. Procédé selon la revendication 1, caractérisé en ce que la fixation de l'ADN se fait par adsorption sur un support solide tel une plaque de microtitration ou une membrane.

3. Procédé selon la revendication 1, caractérisé en ce que la fixation de l'ADN se fait par adsorption à l'aide d'une substance présentant une affinité particulière pour l'ADN que celle-ci soit elle même directement fixé sur le support, ou qu'elle soit fixée indirectement.

4. Procédé selon la revendication 3, caractérisé en ce que la substance présentant une affinité particulière pour l'ADN à l'aide de laquelle se fait la fixation de l'ADN sur le support est prise dans les groupes des substances contenues dans la gélatine ou des substances cationiques.

5. Procédé selon la revendication 4, caractérisé en ce que la substance présentant une affinité particulière pour l'ADN à l'aide de laquelle se fait la fixation de l'ADN sur le support est représentée par une ou des protéines histoniques, qu'elle que soit leur provenance.

6. Procédé selon la revendication 5, caractérisé en ce que la fixation de l'ADN sur le support est réalisée après sensibilisation du support solide par incubation d'un mélange de protéines histoniques en tampon carbonate 0,05-0,1M pH environ 9,6, plusieurs heures, quelle que soit la température d'incubation.

7. Procédé selon l'une des revendications 1 à 6 caractérisé en ce que l'adsorption de l'ADN présent dans l'échantillon est réalisée au cours d'une incubation de 15 à 120 minutes quelle que soit la température d'incubation.

8. Procédé selon l'une des revendications 2 à 7, caractérisé en ce que le support solide subit un ou plusieurs lavages avant le marquage de l'ADN adsorbé.

9. Procédé selon la revendication 8, caractérisé en ce que le support solide subit un ou plusieurs lavages en tampon Tris 2,5 mM pH8, EDTA 1mM, avant le marquage de l'ADN adsorbé.

10. Procédé selon la revendication 9, caractérisé en ce que le marquage de l'ADN adsorbé se fait à l'aide d'enzymes spécifiques de l'ADN.

11. Procédé selon la revendication 10, caractérisé en ce que le marquage de l'ADN est réalisé à l'aide d'une réaction de déplacement de coupure haplotomique ("Nick translation") ou à l'aide de toute autre technique de marquage de l'ADN tels un "marquage par amorce" ou un "marquage terminal" par incorporation, au cours d'une incubation de 15 à 240 minutes, à température de 15 à 40°Celsius environ, d'au moins un désoxyribonucléotide marqué.

12. Procédé selon la revendication 11, caractérisé en ce que le ou les nucléotides incorporés dans l'ADN sont marqués par la biotine.et.l'enzyme utilisé pour sa ou leur détection est lié à l'avidine ou à la streptavidine.

13. Procédé selon la revendication 11 ou 12, caractérisé en ce que le marquage par déplacement de coupure haplotomique est effectué par incubation de l'ADN adsorbé sur le support solide avec 10 à 100 picomoles environ d'un ou plusieurs désoxyribonucléotides triphosphates marqués, éventuellement environ 10 à 100 picomoles de désoxyribonucléotides triphosphates non marqués, environ 0,04 unités d'ADN polymérase et environ 4 picogrammes de désoxyribonucléase I, en présence d'un tampon tel que le Tris-hydroxyri-aminométhane (0,5-5mM), de chlorure de magnésium (2-5mM), et éventuellement de bêta-mercapto-éthanol(7-15mM), et d'albumine bovine sérique (20-60 microgrammes/ml).

14. Procédé selon l'une des revendications 2 à 13, caractérisé en ce que, après marquage de l'ADN, le support solide subit un ou plusieurs lavages avant l'étape de révélation du marqueur incorporé quantitativement dans l'ADN.

15. Procédé selon la revendications 14, caractérisé en ce que, après marquage de l'ADN adsorbé sur le support solide, le ou les lavage(s) sont effectués en tampon Phosphate 0,2M Citrate 0,1M pH 5,4.

16. Procédé selon l'une des revendications 1 à 15 caractérisé en ce que la révélation du marqueur incorporé dans l'ADN se fait à l'aide d'une réaction enzymatique.

17. Procédé selon la revendication 16 caractérisé en ce que le marqueur incorporé est la biotine et que la révélation de ce marqueur incorporé dans l'ADN se fait par addition d'un composé avidine/enzyme ou streptavidine/enzyme, dénommé conjugué.

18. Procédé selon la revendication 17 caractérisé en ce que l'enzyme couplé à l'avidine ou à la streptavidine est de la peroxydase.

19. Procédé selon la revendication 18 caractérisé en ce que la peroxydase couplée à l'avidine ou à la streptavidine est préalablement diluée à la concentration adéquate en tampon Phosphate 0,2M Citrate 0,1M pH 5,4 4% polyéthylène glycol (6000).

20. Procédé selon la revendication 19 caractérisé en ce que la peroxydase couplée à l'avidine ou à la streptavidine est incubé 5 à 30 minutes à température d'environ 4 à 37°Celsius en présence de l'ADN marqué à la biotine.

21. Procédé selon l'une des revendications 17 à 20 caractérisé en ce que, après incubation du conjugué, le support solide subit un ou plusieurs lavages.

22. Procédé selon la revendication 21 caractérisé en ce que, après incubation du conjugué, le ou les lavages du support solide sont réalisés en tampon Phosphate 0,2M Citrate 0,1M pH 5,4.

23. Procédé selon l'une des revendications 16 à 22 caractérisé en ce que la présence du conjugué est révélée à l'aide de tout substrat dont la réaction en présence du conjugué est responsable de l'émission d'un signal quantifiable à l'aide d'un appareillage approprié tel qu'un spectophotomètre, un spectrofluorimètre ou un biocapteur en particulier.

24. Procédé selon l'une des revendications 18 à 23 caractérisé en ce que l'activité enzymatique de la peroxydase est déterminée quantitativement par addition de 3 3′ 5 5′ tétraméthyl benzidine qui sous l'effet de la peroxydase donne une réaction colorée.

25. Procédé selon la revendication 24 caractérisé en ce que la dite réaction colorée est l'objet d'une lecture effectuée quelques minutes plus tard après arrêt de la réaction par addition d'acide sulfurique 2-4N, à l'aide d'un spectrophotomètre à 450 nm.

26. Procédé selon l'une des revendications 8, 14 et 21 caractérisé en ce que le ou les lavages sont effectués en tampon Borate environ 0,1 de force ionique, pH 8,3.

## Patentansprüche

1. Verfahren zum Dosieren von extrazellular in einem Milieu vorhandener DNS, dadurch gekennzeichnet, daß es folgende Schritte umfaßt:
- Fixierung der in dem Milieu vorhandenen DNS durch Adsorption an einem festen Träger,
- Einbauen wenigstens eines Desoxyribonukleotids, das zum Markieren besagter fixierter DNS markiert ist, in die fixierte DNS und
- direktes oder indirektes Entdecken des in besagte fixierte DNS eingebauten Markierers.

2. Verfahren nach Anspruch 1, dadurch gekennzeichnet, daß die Fixierung der DNS durch Adsorption an einem festen Träger wie einer Mikrotitrationsplatte oder einer Membran erfolgt.

3. Verfahren nach Anspruch 1, dadurch gekennzeichnet, daß die Fixierung der DNS durch Adsorption mittels einer Substanz erfolgt, die eine besondere Affinität für die DNS besitzt und selbst direkt oder indirekt an dem Träger befestigt ist.

4. Verfahren nach Anspruch 3, dadurch gekennzeichnet, daß die Substanz mit besonderer Affinität für die DNS, mit deren Hilfe die Fixierung der DNS auf dem Träger erfolgt, aus den Gruppen von Substanzen, die in Gelatine enthalten sind, oder von kationischen Substanzen genommen wird.

5. Verfahren nach Anspruch 4, dadurch gekennzeichnet, daß die Substanz mit besonderer Affinität für die DNS, mit deren Hilfe die Fixierung der DNS auf dem Träger erfolgt, durch ein oder mehrere histonische Proteine, unabhängig von ihrer Herkunft, repräsentiert wird.

6. Verfahren nach Anspruch 5, dadurch gekennzeichnet, daß die Fixierung der DNS auf dem Träger nach Sensibilisierung des festen Trägers durch Inkubation einer Mischung von histonischen Proteinen in Carbonatpuffer 0,05-0,1 M pH etwa 9,6 während mehrerer Stunden, unabhängig von den Inkubationstemperatur, realisiert wird.

7. Verfahren nach einem der Ansprüche 1 bis 6, dadurch gekennzeichnet, daß die Adsorption der DNS, die in der Probe enthalten ist, im Verlauf einer Inkubation von 15 bis 120 min unabhängig von der Inkubationstemperatur vorgenommen wird.

8. Verfahren nach einem der Ansprüche 2 bis 7, dadurch gekennzeichnet, daß der feste Träger einer oder mehreren Waschungen vor dem Markieren der adsorbierten DNS unterworfen wird.

9. Verfahren nach Anspruch 8, dadurch gekennzeichnet, daß der feste Träger einer oder mehreren Waschungen im Puffer Tris 2,5 mM pH 8, EDTA 1 mM vor dem Markieren der adsorbierten DNS unterworfen wird.

10. Verfahren nach Anspruch 9, dadurch gekennzeichnet, daß die Markierung der adsorbierten DNS mittels spezifischer Enzyme der DNS vorgenommen wird.

11. Verfahren nach Anspruch 10, dadurch gekennzeichnet, daß die Markierung der DNS mittels einer Reaktion zur Verschiebung des haplotomischen Schnitts ("Nick translation") oder mittels jeder anderen Markierunstechnik für DNS solche als "Markierung durch Ansatz" oder "terminale Markierung", durch einbauen, im Verlauf einer Inkubation von 15 bis 240 min bei einer Temperatur von etwa 15 bis 40°C, wenigstens einem markierten Desoxyribonucleotid vorgenommen wird.

12. Verfahren nach Anspruch 11, dadurch gekennzeichnet, daß das oder die in die DNS eingebauten Nucleotide durch Biotin markiert und das zu seiner oder ihrer Feststellung verwendete Enzym an Avidin oder an Streptavidine gebunden wird.

13. Verfahren nach Anspruch 11 oder 12, dadurch gekennzeichnet, daß die Markierung durch Verschiebung des haplotomischen Schnitts durch Inkubation der auf dem festen Träger adsorbierten DNS mit etwa 10 bis 100 Picomol eines oder mehrerer markierter triphospahitischer Desoxyribonucleotide, gegebenenfalls etwa 10 bis 100 Picomol nichtmarkierter triphospahitischer Desoxyribonucleotide, etwa 0,04 Einheiten DNS-Polymerase und etwa 4 Picogramm Desoxyribonuclease 1 in Anwesenheit eines Puffers wie Trishydroxyriaminomethan (0,5-5mM), Magnesiumchlorid (2-5mM) und gegebenenfalls β-Mercaptoethanol (7-15mM) und Albumin aus Rinderserum (20-60 µg/ml) vorgenommen wird.

14. Verfahren nach einem der Ansprüche 2 bis 13, dadurch gekennzeichnet, daß nach dem Markieren der DNS der feste Träger einer oder mehreren Waschungen vor dem Schritt des Entdeckens des quantitativ in die DNS eingebauten Markierers unterworfen wird.

15. Verfahren nach Anspruch 14, dadurch gekennzeichnet, daß nach dem Markieren der an dem festen Träger adsorbierten DNS die Waschung(en) in einem Puffer aus Phosphat 0,2M Citrat 0,1M ph 5,4 durchgeführt wird/werden.

16. Verfahren nach einem der Ansprüche 1 bis 15, dadurch gekennzeihnet, daß das Entdecken des in die DNS eingebauten Markierers mittels einer enzymatischen Reaktion vorgenommen wird.

17. Verfahren nach Anspruch 16, dadurch gekennzeichnet, daß der eingebaute Markierer Biotin ist und daß das Entdecken dieses in die DNS eingebauten Markierers durch Zusatz einer Verbindung Avidin/Enzym oder Streptavidin/Enzym, als gekoppelt bezeichnet, vorgenommen wird.

18. Verfahren nach Anspruch 17, dadurch gekennzeichnet, daß das an das Avidin oder das Streptavidin gekoppelte Enzym Peroxydase ist.

19. Verfahren nach Anspruch 18, dadurch gekennzeichnet, daß die an das Avidin oder Streptavidin gekoppelte Peroxydase vorher auf eine adäquate Konzentration im Puffer Phosphat 0,2M Citrate 0,1M pH 5,4 4% Polyethylenglykol (6000) verdünnt wird.

20. Verfahren nach Anspruch 19, dadurch gekennzeichnet, daß die an das Avidin oder Streptavidin gekoppelte Peroxydase 5 bis 30 min bei einer Temperatur von etwa 4 bis 37°C in Anwesenheit der durch das Biotin markierten DNS inkubiert wird.

21. Verfahren nach einem der Ansprüche 17 bis 20, dadurch gekennzeichnet, daß nach Inkubation der gekoppelten Verbindung der feste Träger einer oder mehreren Waschungen unterworfen wird.

22. Verfahren nach Anspruch 21, dadurch gekennzeichnet, daß nach Inkubation der gekoppelten Verbindung die Waschung(en) des festen Trägers im Puffer Phosphat 0,2M Citrat 0,1M pH 5,4 durchgeführt wird/werden.

23. Verfahren nach einem der Ansprüche 16 bis 22, dadurch gekennzeichnet, daß die Anwesenheit der gekoppelten Verbindung mittels jeden Substrats festgestellt wird, dessen Reaktion in Anwesenheit der gekoppelten Verbindung für die Emission eines durch eine geeignete Apparatur wie ein Spektrophotometer, ein Spektrofluorimeter oder insbesondere einen Biodetektor quantifizierbaren Signals verantwortlich ist.

24. Verfahren nach einem der Ansprüche 18 bis 23, dadurch gekennzeichnet, daß die enzymatische Aktivität der Peroxydase quantitativ durch Zusatz von 3 3' 5 5' - Tetramethylbezidin bestimmt wird, das unter der Einwirkung der Peroxydase eine Farbreaktion zeigt.

25. Verfahren nach Anspruch 24, dadurch gekennzeichnet, daß die besagte Farbreaktion Gegenstand einer Ablesung ist, die einige Minuten nach den Anhalten der Reaktion durch Zusatz von 2-4n Schwefelsäure mittels eines Spektrophotometers bei 450 nm vorgenommen wird.

26. Verfahren nach einem der Ansprüche 8, 14 und 21, dadurch gekennzeichnet, daß die Waschung(en) im Puffer Borat mit etwa 0,1 ionischer Kraft, pH 8,3 vorgenommen wird/werden.

## Claims

1. A method of metering DNA which is present in an extra-cellular position in a medium,
characterised in that it comprises the following stages:
- fixing the DNA present in the medium by adsorption on a solid carrier,
- incorporation of at least one marked deoxyribonucleotide in the fixed DNA to mark said fixed DNA, and
- direct or indirect revelation of the marker incorporated in the fixed DNA.

2. A method according to claim 2, characterised in that the DNA is fixed by adsorption on a solid carrier such as a microtitration plate or a membrane.

3. A method according to claim 2, characterised in that the DNA is fixed through adsorption by means of a substance with a particular affinity for DNA, the substance itself being either directly fixed or indirectly fixed on the carrier.

4. A method according to claim 4, characterised in that the substance which has a particular affinity for DNA and by means of which the DNA is fixed on the carrier is taken from the groups of substances contained in gelatin or cationic substances.

5. A method according to claim 5, characterised in that the substance which has a particular affinity for DNA and by means of which the DNA is fixed on the carrier is represented by one or more histones from any source.

6. A method according to claim 6, characterised in that the DNA is fixed on the carrier after sensitisation of the solid carrier through incubation of a mixture of histones in a 0.05-0.1 M carbonate buffer, pH approximately 9.6, for some hours at any incubation temperature.

7. A method according to any of claims 2 to 7, characterised in that the DNA present in the specimen is adsorbed in the course of a 15 to 120-minute incubation process at any incubation temperature.

8. A method according to any of claims 3 to 8, characterised in that the solid carrier is washed one or more times before the marking of the adsorbed DNA.

9. A method according to claim 9, characterised in that the solid carrier is washed one or more times in a 2.5mM Tris, pH 8, ImM EDTA buffer before the marking of the adsorbed DNA.

10. A method according to claim 10, characterised in that the adsorbed DNA is marked by means of DNA-specific enzymes.

11. A method according to claim 11, characterised in that the DNA is marked by means of a haplotomic cut displacement reaction ('nick translation') or any other DNA marking technique such as 'marking by primer' or 'end marking', through incorporation of at least one marked deoxyribonucleotide in the course of a 15 to 240-minute incubation at a temperature of approximately 15 to 40° Celsius.

12. A method according to claim 12, characterised in that the nucleotide or nucleotides incorporated in the DNA are marked by biotin, and the enzyme used to detect it or them is bonded to avidin or streptavidin.

13. A method according to claim 12 or 13, characterised in that the marking by haplotomic cut displacement is effected by incubating the DNA adsorbed on the solid carrier with approximately 10 to 100 picomoles of one or more marked triphosphate deoxyribonucleotides. possibly approximately 10 to 100 picomoles of non-marked triphosphate deoxyribonucleotides, approximately 0.04 unit of DNA polymerase and approximately 4 picograms of deoxyribonuclease 1, in the presence of a buffer such as tris-hydroxyri-aminomethane (0.5-5mM), and in the presence of magnesium chloride (2-5mM), possibly of beta-mercaptoethanol (7-15mM), and of bovine serum albumin (20-60 micrograms/ml).

14. A method according to any of claims 3 to 14, characterised in that after the marking of the DNA the solid carrier is washed one or more times before the stage when the marker incorporated quantitatively in the DNA is revealed.

15. A method according to claim 15, characterised in that after the marking of the DNA adsorbed on the solid carrier the wash or washes are effected in a 0.2M phosphate, 0.1M citrate, pH5.4 buffer.

16. A method according to any of claims 1 to 16, characterised in that the marker incorporated in the DNA is revealed by means of an enzyme reaction.

17. A method according to claim 17, characterised in that the incorporated marker is biotin, and this marker incorporated in the DNA is revealed by adding an avidin/enzyme or streptavidin/enzyme composition described as a conjugate.

18. A method according to claim 18, characterised in that the enzyme coupled to avidin or streptavidin is peroxidase.

19. A method according to claim 19, characterised in that the peroxidase coupled to the avidin or streptavidin is previously diluted to the appropriate concentration in a 0.2M phosphate, 0.1 M citrate, pH 5.4, 4% polyethylene glycol (6000) buffer.

20. A method according to claim 20, characterised in that the peroxidase coupled to the avidin or streptavidin is incubated for 5 to 30 minutes at a temperature of approximately 4 to 37° Celsius in the presence of the biotin-marked DNA.

21. A method according to any of claims 18 to 21, characterised in that the solid carrier undergoes one or more washes after the incubation of the conjugate.

22. A method according to claim 22, characterised in that the wash or washes of the solid carrier are carried out in a 0.2M phosphate, 0.1M citrate, pH 5.4 buffer after the incubation of the conjugate.

23. A method according to any of claims 17 to 23, characterised in that the presence of the conjugate is revealed by means of any substrate, the reaction of which in the presence of the conjugate is responsible for transmitting a signal which can be quantified by means of an appropriate apparatus such as a spectrophotometer, a spectrofluorimeter or particularly a bio-sensor.

24. A method according to any of claims 19 to 24, characterised in that the enzymatic activity of the peroxidase is determined quantitatively by adding 3 3' 5 5' tetramethyl benzidine, which gives a coloured reaction by the action of the peroxidase.

25. A method according to claim 25, characterised in that said coloured reaction is the subject of a reading taken some minutes later when the reaction has stopped, by the addition of 2-4N sulphuric acid, using a 450nm spectrophotometer.

26. A method according to claim 9, 15 or 22, characterised in that the wash or washes are effected in a borate buffer around 0.1 ionic strength and pH 8.3.
